Europäisches Patentamt

European Patent Office

Office européen des brevets

(19)

(11) Publication number: **0 483 614 B1**

(12) **EUROPEAN PATENT SPECIFICATION**

(45) Date of publication of patent specification: **21.06.95** (51) Int. Cl.⁶: **A61K 31/195**

(21) Application number: **91117799.6**

(22) Date of filing: **18.10.91**

(54) Use of amino acids for the protection of and metabolic recovery of ischemic cardiac tissue.

(30) Priority: **30.10.90 US 605514**

(43) Date of publication of application:
**06.05.92 Bulletin 92/19**

(45) Publication of the grant of the patent:
**21.06.95 Bulletin 95/25**

(84) Designated Contracting States:
**AT BE CH DE DK ES FR GB GR IT LI LU NL SE**

(56) References cited:
**WO-A-88/01872**
**DE-A- 3 228 127**

**ADVANCES IN SHOCK RESEARCH, vol. 8, 1982, pages 163-170, Alan R. Liss, Inc.,New York, US; A.M. DANIEL et al.: "Exogenous amino acids as fuel in shock"**

**J. AMER. DIET. ASSOC., vol. 87, no. 9, September 1987, pages 1202-1206; C.J.C.GRIMES et al.: "The effect of preoperative total parenteral nutrition onsurgery outcomes"**

(73) Proprietor: **CLINTEC NUTRITION COMPANY**
**Three Parkway North,**
**Suite 500**
**Deerfield,**
**Illinois 60015 (US)**

(72) Inventor: **Madsen, David**
**600 Ardmore Terrace**
**Libertyville, Ill. 60048 (US)**
Inventor: **Pace, Gary**
**23 Reagent Wood Road**
**Northfrield, Ill. 60093 (US)**
Inventor: **Rowe, Bruce**
**229 Main Street**
**Evanston, Ill. 60202 (US)**

(74) Representative: **Vuille, Roman et al**
**Avenue Nestlé 55**
**CH-1800 Vevey (CH)**

Rank Xerox (UK) Business Services
(3.10/3.09/3.3.3)

## EP 0 483 614 B1

**Description**

BACKGROUND OF THE INVENTION

The present invention relates generally to compositions for supplying nutrients to cardiac tissue. More specifically, the present invention relates to the preparation of a medicament solution that can be used for cardiac therapy.

Ischemia refers to a condition wherein tissue suffers from a decrease or cessation of perfusion by blood. Ischemia can occur due to a blockage of the blood vessels, for example, due to atherosclerosis wherein there is a narrowing of the diameter of the vessel.

Cardiac tissue is susceptible to ischemia and can be severely damaged by such an event. Cardiac ischemia can be caused by a number of events. The recovery from cardiac ischemia is critical in order to insure proper cardiac function, and in many cases survival of the patient. Cardiac ischemic events can be caused, for example, by heart attack or other disease states or from therapeutic intervention such as angioplasty or bypass surgery. Such ischemic events can either be minimal or extensive.

During and after a heart attack, one of the principal goals is to limit the damage caused by the resulting ischemic event. This is critical to a patient's recovery.

Although ischemic events can be the result of a disease state and therefore not entirely predictable, there are times when an ischemic event can be predicted. Indeed, some therapeutic interventions can result in an ischemic event.

For example, it is known to use percutaneous transluminal coronary angioplasty, hereinafter referred to as "angioplasty", to increase the diameter of occluded arteries. Generally, angioplasty is performed using a multilumen inflatable balloon catheter. According to a typical procedure, the catheter is directed to an area wherein an inflatable balloon portion is located in juxtaposition to the occluded portion of the artery. When the catheter is appropriately positioned, so that it is surrounded by an occluding atheroma, the lumen is inflated. As the balloon expands, it deforms the occluding atheroma adjacent to its surface. This creates a larger inner diameter within the occluded artery. The usual method is to repeat the inflation an arbitrary number of times. It is hoped that by repeatedly deforming the occluding atheromas, they will become permanently deformed thereby providing the affected artery with a lumen having a sufficiently large inner diameter.

Further, it is also believed that prolonging the duration that the balloon is inflated reduces the possibility of re-stenosis. By reducing the rate at which the balloon is inflated, the atheroma is slowly stretched and deformed. It is believed that the combination of these actions reduces the likelihood of re-stenosis. The balloon is then collapsed and retracted.

Standard angioplasty protocol requires that the balloon is inflated for a period of about 60 seconds. During this period, the flow of blood through the artery is blocked. Accordingly, the supply of blood perfusing the tissue past the blockage becomes inadequate to sustain cellular metabolism. This creates ischemia. If the ischemic episode is prolonged, the affected tissue will die, a condition known as infarction.

When angioplasty is performed to reduce occlusion in a coronary artery, i.e., percutaneous transluminal coronary angioplasty, myocardial tissue downstream from the inflated balloon becomes ischemic. The extent of the myocardial tissue effected and morbidity of the corresponding pathological syndrome depends greatly on the length of time the blood flow is interrupted.

SUMMARY OF THE INVENTION

The present invention provides for the preparation of a compositon for improving cardiac function that can be used as a cardiac therapy in which pre-existing ischemia is being treated or in which ischemia may result from a therapeutic intervention, making use of an amino acid solution that is infused into a patient. WO-A-80/01872 relates to the use of amino acid solutions to treat atherosclerosis, as opposed to cardiac therapy. Specifically, it relates to the use of amino acid solutions in an attempt to lower plasma cholesterol levels and arrest, reverse and, to a certain extent, cure the arterial plaque deposition and degenerative vascular wall changes associated with atherosclerosis.

The purpose of the present invention is to provide a composition useful for the treatment of either preexisting cardiac ischemia or ischemia which can result from a therapeutic intervention e.g. The invention provides an amino acid solution which can be used as an adjunct with current cardiac therapies in which a pre-existing ischemia is being treated. On the other hand, if desired, the amino acid solution can be utilized prior to an anticipated ischemia, for example, an ischemic event caused by a therapeutic intervention. The amino acid solution of the present invention provides improved cardiac

2

function after an ischemic insult.

To this end, the present invention provides the use of amino acids in the manufacture of a composition for treating cardiac ischemia having a therapeutically efficient amount of amino acids.

In an embodiment of the present invention, the solution is enriched in the amino acids that are metabolized to $CO_2$ and $H_2O$ by pathways common to fatty acid oxidation such as leucine, isoleucine, valine, methionine, phenylalanine, tyrosine, and tryptophan.

In an embodiment, the solution includes amino acids that supply intermediates to the "TCA" cycle for maintaining oxidative metabolism, such as glutamic acid, glutamine, aspartic acid, and asparagine.

In an embodiment, the proportion and type of amino acids that are administered during the therapy are modified.

In an embodiment of the use, the amino acids are chosen from the group consisting of: arginine; leucine; isoleucine; lysine; valine; phenylalanine; histidine; threonine; methionine; tryptophan; alanine; proline; serine; tyrosine; amino acetic acid; isoleucine; leucine; and valine.

In an embodiment of the use, the amino acids comprise: isoleucine; leucine; and valine.

In an embodiment of the present invention, amino acids are used to manufacture a composition that is administered to a patient prior to an expected ischemic event.

Additional features and advantages of the present invention are described in, and will be apparent from, the detailed description of the presently preferred embodiments.

## DETAILED DESCRIPTION OF THE PRESENTLY PREFERRED EMBODIMENTS

The present invention provides the use of amino acids to manufacture a composition for improving cardiac function that can be used in conjunction with any cardiac therapy for treating a pre-existing ischemia such as thrombolytic therapy. The composition can therefore be used as an adjunct to traditional cardiac therapy for improving cardiac function after an ischemic event. The composition includes a therapeutically effective amount of an amino acid composition.

Additionally, the present invention provides for the use of amino acids to manufacture a composition for improving cardiac function and limiting or preventing damage from an expected ischemic event. To this end, the invention provides a composition that can be administered prior to and/or after an expected ischemic event, for example, prior to a therapeutic intervention, e.g., bypass surgery or angioplasty.

It has been found that a therapeutically effective amount of an amino acid solution, either given parenterally or enterally, will improve cardiac function after an ischemic insult.

In an embodiment, an amino acid solution including: arginine; leucine; isoleucine; lysine; valine; phenylalanine; histidine; threonine; methionine; tryptophan; alanine; proline; serine; tyrosine; and amino-acetic acid is administered to a patient who has experienced an ischemic event, or is expected to. An example of an amino acid solution formulation that will function satisfactorily is 10% TRAVASOL® (Amino Acid) Injection distributed by Clintec Nutrition Company, Deerfield, IL.

In an embodiment, an amino acid solution including the branched chain amino acids: isoleucine, leucine, and valine is administered to a patient who has experienced an ischemic event, or is expected to. Preferably, the branched chain amino acids are included in approximately a 1:1:1 molar ratio. An example of such a branched chain amino acid formula is 4% BRANCHAMIN® (branched chain amino acid) Injection distributed by Clintec Nutrition Company, Deerfield, IL.

In an embodiment, an amino acid solution including: glutamic acid; aspartic acid; arginine; leucine; isoleucine; lysine; valine; phenylalanine; histidine; threonine; methionine; tryptophan; alanine; proline; serine; tyrosine; and amino-acetic acid is administered to a patient who has experienced an ischemic event, or is expected to. An example of such an amino acid formulation is Novamine™ Amino Acids Injection distributed by Clintec Nutrition Company, Deerfield, IL.

In an embodiment, the amino acid solution includes: arginine; leucine; isoleucine; lysine; valine; phenylalanine; histidine; threonine; methionine; tryptophan; alanine; proline; serine; tyrosine; amino acetic acid; with the branched chain amino acid comprising approximately 50% (w:w) of the total amino acid. The branched chain amino acids can comprise 15 to 55% by weight of the total amino acid composition.

Depending on the therapy or treatment, the amino acid solution can be modified. For example, the amino acid solution can either be balanced, or can be enriched in those amino acids that are metabolized to $CO_2$ and $H_2O$ by pathways also used in fatty acid oxidation. Such amino acids include leucine, isoleucine, valine, methionine, phenylalanine, tyrosine, and tryptophan. Similarly, those amino acids that supply intermediates to the TCA ("KREBS") cycle are useful in maintaining the oxidative metabolism during ischemia. Examples of these amino acids include glutamic acid, glutamine, aspartic acid, and asparagine.

3

It may be desirable to modify the composition of the amino acid solution during the various phases of therapy such as during ischemia and during recovery from ischemia. Accordingly, one or more different composition can be utilized and have specific benefits as an adjunct at different stages of the therapy.

By way of example, and not limitation, experimental designs incorporating the present invention will now be given:

Experimental Design:

Rat hearts were perfused in a non-recirculating retrograde (i.e., Langendorff) manner for 10 minutes before switching to an anterograde working heart mode for 25 minutes at which time baseline hemodynamics were recorded. The hearts were then subjected to 20.5 minutes of global no-flow ischemia (NFI). This was followed by 40 minutes of reperfusion in the working heart mode. Hearts were re-perfused with all solutions at 12-13 ml per minute.

Previous studies have demonstrated that peak recovery of hemodynamic function occurs at approximately 40 minutes reperfusion at this severity of ischemic challenge. The preload was set at 15 cm $H_2O$, the afterload at 80 cm $H_2O$ and the hearts were paced at 315 beats per minutes by a 2.2 Volt, 1.6 msec duration atrial stimulus.

All hearts were perfused with 11 mM Glucose in KHB. Additionally, the hearts were perfused, in the following groups, with the following solutions:

Group #1   Aerobic controls: Travasol® (T) (from Baxter Healthcare, Deerfield, IL) present throughout(t) at

Group #2   NFI: Travasol® present throughout [Tt]

Group #3   NFI: Travasol® throughout + BranchAmin® (from Baxter Healthcare, Deerfield, IL) reperfusion only [Tt + Br]

Group #4   NFI: Non-Treated [0], i.e., ischemic controls

Group #5   NFI: BranchAmin® (B), reperfusion only(r)[Br]

Group #6   NFI: Travasol®, reperfusion only [Tr]

Group #7   NFI: BranchAmin®, throughout [Bt]

Group #8   NFI: Travasol® and BranchAmin® present throughout [T + B]t

The Following Hemodynamic Parameters Were Measured:

1. Coronary flow [CF] ml/min [Cardiac perfusion]

2. Aortic flow [AO] ml/min [Systemic outflow]

3. Cardiac output [CO] ml/min [Total output]

4. Left Ventricular minute work [LVMW] $g^*m$/min [cardiac work]

5. Left Ventricular Systolic Pressure [LVSP] mmHg [peak developed pressure, contractile apparatus performance]

6. Left Ventricular Diastolic Pressure [LVDP] mmHg (main resistive element to subendocardial perfusion)

7. Aortic Systolic Pressure [ASP] mmHg

8. Aortic Diastolic Pressure [ADP] mmHg (major determinant of coronary driving pressure]

9. Mean Aortic Pressure [MAP] mmHg [afterload]

10. Coronary Vascular Resistance [CVR] $mmHg^*$min/ml (indicator of coronary patency e.g. elevated by osmotic swelling compression of the microvasculature by lysed cellular debris emboli)

11. Rate Pressure Product [RPP] mmHg/min [myocardial oxygen consumption Index]

12. Cardiac Efficiency Index [EFFI] $g^*m$/mmHg [defined as work/O2 consumed (i.e., EFFI = LVMW/RPP) indicates cardiac pump efficiency and oxygen wastage]

13. Recovery Time [RecT] min [duration of reperfusion required before an aortic outflow at 80 cm H2O is produced]

14. Initial Ischemic Mean Left Ventricular Pressure [LVP i] mmHg

15. Peak Ischemic Mean Left Ventricular Pressure [LVPmax i] mmHg

16. Final Ischemic Mean Left Ventricular Pressure [LVPf i] mmHg

17. Peak Amplitude Ischemic Contracture [ALVPpki] [contracture severity]

18. Post Peak Contracture Relaxation [ALVP post i] [contracture waning due to passive stretch from cytoskeletal distortion/disintegration]

19. Last beat, min [latency to arrest, oxygen reserve]

20. Latency to contracture onset [$St^*Cont$] min [contracture onset delay]

21. Latency to contracture peak [Pk Cont] min [contracture onset delay]

Statistics were based on the two-tail Student test (p < 0.5) or Mann-Whitney U Test (Rec T) Only.

The aerobic control group demonstrated a gradual increase in aortic flow, cardiac output, left ventricular min work, and cardiac efficiency index throughout the 70 min period while other hemodynamic parameters remained unaltered. This demonstrated that the preparation was stable throughout the time range considered in this study.

Pre-ischemic hemodynamics recorded in this investigation were similar to those recorded for healthy in vivo subjects demonstrating the accuracy of the study.

Experimental conditions and pre-ischemic hemodynamics are present for each experimental group in Table 1 (see below). No apparent differences exist between groups.

The results of the experiments were as follows.

Both Travasol® and BranchAmin® did not significantly alter baseline hemodynamics in the isolated rat heart (see Table 2).

With respect to Ischemic contracture modulation by Travasol® and BranchAmin® neither agent significantly altered ischemic contracture severity nor time course (see Table 2). There existed a trend for the delay in onset of contracture by both Travasol® and BranchAmin®.

Both Travasol® and BranchAmin® treated hearts demonstrated significantly enhanced recovery of hemodynamic functions when compared to non-treated ischemic hearts (group 4; see Table 3). At 40 minutes reperfusion time this enhancement included the entire spectrum of hemodynamic parameters investigated with the frequent exception of mean aortic pressure (MAP) and left ventricular end diastolic pressure (LVDP). This effect was observed in all drug treatment regimes. This pattern of enhanced recovery of a broad spectrum of hemodynamic functions indicates that the mechanism involves global myocardial salvaging.

Neither Travasol® nor BranchAmin®, in any regime, were able to restore any hemodynamic function (i.e., except occasional MAP and LVDP) to pre-ischemic levels (see Table 3); compare groups 2-8 with aerobic group #1. Therefore, 20.5 minutes no-flow ischemia produced subpopulations of myocardium that were either: a) infarcted during ischemia; b) condemned at reperfusion onset; or c) non-salvageable by Travasol® or BranchAmin® intervention.

For both Travasol® and BranchAmin®, no statistically significant differences in evoked cardioprotective effectiveness could be found when the drug was present throughout the experiment versus when the drug was present during the reperfusion period exclusively. This indicates that the effective period of action for both drugs occurs primarily during reperfusion. (Table 3: compare groups 2 versus 6; and 5 versus 7.)

Travasol® was found to be slightly more efficacious than BranchAmin® in enhancing post-ischemic hemodynamic recovery. These effects were parameter-selective and reached statistically significant levels during later reperfusion in drug present in the "throughout" paradigms only (see Table 3; group 2 versus 7). The parameters most influenced were developed pressures (i.e., LVSP, LVDP, ASP), contractility (LVdP/dT), and myocardial oxygen consumption (RPP). Collectively these parameters suggest enhanced salvation of the contractile apparatus.

Post-ischemic hemodynamic recovery enhancement in hearts treated with a mixture of Travasol® and BranchAmin® (group 8) is significantly greater than those treated with BranchAmin® only (group 7), but not significantly greater than those treated with Travasol® only (group 2) (see Table 3). This suggests that either Travasol® contains additional cardioprotective agents which are absent in BranchAmin® or that the levels of branched amino acids present in Travasol® (i.e., 1.2mM) are sufficient to evoke cardioprotection similar to BranchAmin's concentration.

The co-treatment with a mixture of Travasol® and BranchAmin® significantly enhanced recovery over Travasol-treatment (i.e., group 2) only at 30 min reperfusion of the following parameters: LVSP; LVdP/dT; and RPP. This was true only when the BranchAmin® co-treatment was present throughout.

In contrast, co-treatment elicited significant enhanced recovery of CF, AO, LVMW, LVdT, RPP, and EFFI over BranchAmin-treatment. The latter pattern suggests that co-treatment enhances myocardial salvaging over BranchAmin® treatment alone.

## TABLE 1 PROTOCOL GROUP EQUIVALENCE

| Protocol | 1 | 2 | 3 | 4 |
|---|---|---|---|---|
| Substrate | TL | TL | TL | 0 |
| n | 10 | 15 | 13 | 11 |
| | MEAN $\pm$ SEM | MEAN $\pm$ SEM | MEAN $\pm$ SEM | MEAN $\pm$ SEM |
| Temp $^{\circ}$C | 36.97 $\pm$ 0.04 | 37.06 $\pm$ 0.03 | 37.02 $\pm$ 0.04 | 37.0 $\pm$ 0.04 |
| Body wt | 329 $\pm$ 7 | 319 $\pm$ 5 | 326 $\pm$ 4 | 332 $\pm$ 4 |
| HR-P | 315 $\pm$ 0 | 315 $\pm$ 0 | 319 $\pm$ 3 | 316 $\pm$ 1 |
| CF-WP | 20.2 $\pm$ 0.5 | 19.8 $\pm$ 0.7 | 21.3 $\pm$ 0.7 | 19.7 $\pm$ 0.7 |
| AO-WP | 70 $\pm$ 1 | 69 $\pm$ 1 | 71 $\pm$ 1 | 68 $\pm$ 2 |
| CO-WP | 90 $\pm$ 1 | 89 $\pm$ 2 | 92 $\pm$ 1 | 88 $\pm$ 3 |
| LVMW-WP | 74 $\pm$ 1 | 73 $\pm$ 1 | 75 $\pm$ 2 | 71 $\pm$ 2 |
| LVSP-WP | 116 $\pm$ 2 | 114 $\pm$ 2 | 114 $\pm$ 2 | 115 $\pm$ 1 |
| LVDP-WP | 11.5 $\pm$ 0.6 | 12.6 $\pm$ 0.4 | 11.4 $\pm$ 0.5 | 10.4 $\pm$ 1.0 |
| LVdP/dl-WP | 2516 $\pm$ 1 | 2614 $\pm$ 1 | 2653 $\pm$ 3 | 2692 $\pm$ 9 |
| ASP-WP | 120 $\pm$ 1 | 120 $\pm$ 1 | 119 $\pm$ 1 | 117 $\pm$ 1 |
| ADP-WP | 30.6 $\pm$ 0.6 | 30.2 $\pm$ 0.4 | 29.8 $\pm$ 0.6 | 30.0 $\pm$ 0.9 |
| MAP-WP | 60.6 $\pm$ 0.6 | 60.1 $\pm$ 0.4 | 59.6 $\pm$ 0.5 | 59.0 $\pm$ 0.4 |
| CVR-WP | 2.4 $\pm$ 0.1 | 2.4 $\pm$ 0.1 | 2.3 $\pm$ 0.1 | 2.5 $\pm$ 0.1 |

| KRPP-WP | 36.4 ± 0.5 | 35.8 ± 0.6 | 36.4 ± 0.5 | 36.5 ± 0.5 |
| LFF1-WP | 2.04 ± 0.03 | 2.03 ± 0.03 | 2.05 ± 0.02 | 1.94 ± 0.05 |

**TABLE 1 (CONT.)**

| Protocol | 5 | 6 | 7 | 8 |
|---|---|---|---|---|
| Substrate | Br | Tr | BT | [TiB]t |
| n | 12 | 8 | 11 | 10 |
| | MEAN ± SEM | MEAN ± SEM | MEAN ± SEM | MEAN ± SEM |
| Temp °C | 37.03 ± 0.03 | 37.05 ± 0.03 | 37.03 ± 0.04 | 37.01 ± 0.02 |
| Body wt | 326 ± 4 | 323 ± 5 | 320 ± 4 | 321 ± 4 |
| HR-P | 316 ± 1 | 315 ± 1 | 318 ± 3 | 315 ± 0 |
| CF-WP | 20.7 ± 0.7 | 20.8 ± 1.1 | 20.7 ± 0.7 | 19.3 ± 0.7 |
| AO-WP | 73 ± 1 | 70 ± 2 | 69 ± 1 | 69 ± 1 |
| CO-WP | 93 ± 2 | 91 ± 2 | 90 ± 1 | 88 ± 1 |
| LVMW-WP | 75 ± 2 | 73 ± 2 | 73 ± 1 | 72 ± 1 |
| LVSP-WP | 114 ± 2 | 115 ± 2 | 113 ± 2 | 117 ± 2 |
| LVDP-WP | 11.6 ± 0.9 | 10.9 ± 1.0 | 11.0 ± 0.8 | 11.1 ± 0.6 |
| LVdP/dl-WP | 2617 ± 9 | 2610 ± 6 | 2701 ± 10 | 2619 ± 2 |
| ASP-WP | 119 ± 1 | 119 ± 2 | 118 ± 1 | 120 ± 1 |
| ADP-WP | 29.0 ± 0.4 | 29.0 ± 0.8 | 30.0 ± 0.6 | 29.8 ± 0.7 |
| MAP-WP | 59.1 ± 0.4 | 59.1 ± 0.6 | 59.3 ± 0.5 | 60 ± 0.6 |
| CVR-WP | 2.3 ± 0.1 | 2.4 ± 0.1 | 2.4 ± 0.1 | 2.6 ± 0.1 |
| KRPP-WP | 36.1 ± 0.6 | 36.2 ± 0.7 | 35.9 ± 0.5 | 36.6 ± 0.6 |
| LFF1-WP | 2.08 ± 0.04 | 2.02 ± 0.03 | 2.03 ± 0.02 | 1.97 ± 0.04 |

## TABLE 2

**The effects of Travasol® and BranchAmin® on pre-Ischemic hemodynamics and ischemic contracture.**

| Substrate | O | T | B | | |
|---|---|---|---|---|---|
| n | 31 | 38 | 11 | P | P |
| | MEAN | MEAN | MEAN | TvsO | BvsO |
| PRE-ISCHEMIC HEMODYNAMICS | | | | | |
| HR-P | 316 $\pm$ 1 | 316 $\pm$ 1 | 318 $\pm$ 3 | NS | NS |
| CF-WP | 20.4 $\pm$ 0.4 | 20.4 $\pm$ 0.4 | 20.7 $\pm$ 0.8 | NS | NS |
| AO-WP | 71 $\pm$ 1 | 70 $\pm$ 1 | 69 $\pm$ 1 | NS | NS |
| CO-WP | 91 $\pm$ 1 | 91 $\pm$ 1 | 90 $\pm$ 1 | NS | NS |
| LVMW-WP | 73 $\pm$ 1 | 74 $\pm$ 1 | 73 $\pm$ 1 | NS | NS |
| LVSP-WP | 115 $\pm$ 1 | 114 $\pm$ 1 | 113 $\pm$ 1 | NS | NS |
| LVDP-WP | 11.0 $\pm$ 0.5 | 11.9 $\pm$ 0.3 | 11.0 $\pm$ 0.8 | NS | NS |
| LVdP/dT-WP | 2649 $\pm$ 31 | 2601 $\pm$ 33 | 2702 $\pm$ 50 | NS | NS |
| ASP-WP | 118.5 $\pm$ 0.8 | 119.8 $\pm$ 0.6 | 117.9 $\pm$ 1.5 | NS | NS |
| ADP-WP | 29.4 $\pm$ 0.4 | 30.2 $\pm$ 0.3 | 30.1 $\pm$ 0.6 | NS | NS |
| MAP-WP | 59.1 $\pm$ 0.2 | 60.0 $\pm$ 0.3 | 59.3 $\pm$ 0.5 | * | NS |
| CVR-WP | 2.40 $\pm$ 0.06 | 2.38 $\pm$ 0.04 | 2.36 $\pm$ 0.09 | NS | NS |
| kRPP-WP | 36.3 $\pm$ 0.3 | 36.2 $\pm$ 0.3 | 35.9 $\pm$ 0.5 | NS | NS |
| EFF1-WP | 2.01 $\pm$ 0.02 | 2.04 $\pm$ 0.02 | 2.03 $\pm$ 0.02 | NS | NS |
| ISCHEMIC CONTRACTURE | | | | | |
| LVP*i | 10.1 $\pm$ 0.2 | 10.2 $\pm$ 0.2 | 9.9 $\pm$ 0.3 | NS | NS |
| LVPmax i | 33 $\pm$ 3 | 35 $\pm$ 3 | 34 $\pm$ 4 | NS | NS |
| LVPf i | 27 $\pm$ 2 | 28 $\pm$ 3 | 29 $\pm$ 4 | NS | NS |
| St* Cont | 14.4 $\pm$ 0.9 | 15.5 $\pm$ 0.5 | 15.6 $\pm$ 1.0 | NS | NS |
| Pk Cont | 17.4 $\pm$ 0.7 | 18.0 $\pm$ 0.4 | 17.8 $\pm$ 0.8 | NS | NS |
| last beat | 116 $\pm$ 4 | 124 $\pm$ 5 | 131 $\pm$ 12 | NS | NS |
| ALVP pk | 23 $\pm$ 3 | 25 $\pm$ 3 | 24 $\pm$ 4 | NS | NS |
| ALVP post | -7 $\pm$ 2 | -7 $\pm$ 1 | -8 $\pm$ 4 | NS | NS |

NS = Non Significant ($p \geq 0.05$)

* = Significant ($p \leq 0.05$)

O Non-Treated

T Travasol®-Treated

B BranchAmin®-Treated

## TABLE 3

**COMPARISON OF % RECOVERY OF HEMODYNAMIC FUNCTIONS BETWEEN AEROBIC (GROUP 1), AND ISCHEMIC (GROUPS 2-8)**

Note: All values in the ischemic/reperfused hearts (Groups 2-8) are significantly lower than those in aerobic hearts (Group 1), except for those marked ns. $p = \leq 0.05$

Protocol

| Group | 1 | 2 | 3 | 4 | 5 | 6 | 7 | 8 |
|---|---|---|---|---|---|---|---|---|
| %CF-10 | 103±1 | 40±8 | 46±9 | 19±5 | 51±10 | 40±13 | 51±9 | 34±8 |
| %CF-20 | 105±2 | 72±5 | 77±4 | 16±4 | 53±8 | 67±8 | 56±10 | 76±2 |
| %CF-30 | 104±2 | 72±4 | 80±5 | 32±9 | 64±6 | 73±8 | 62±8 | 73±3 |
| %AO-10 | 102±1 | 10±6 | 4±2 | 2±2 | 15±5 | 10±7 | 11±5 | 3±3 |
| %AO-20 | 103±1 | 37±7 | 25±5 | 2±2 | 20±7 | 29±7 | 22±7 | 40±6 |
| %AO-30 | 105±1 | 47±7 | 35±5 | 4±4 | 28±7 | 40±8 | 28±8 | 50±6 |
| %AO-40 | 106±1 | 50±6 | 39±6 | 7±5 | 35±7 | 40±7 | 33±8 | 54±5 |
| %CO-10 | 102±1 | 17±6 | 13±3 | 6±3 | 22±6 | 17±8 | 21±6 | 10±4 |
| %CO-20 | 104±1 | 44±6 | 38±4 | 5±2 | 28±7 | 38±7 | 30±8 | 48±5 |
| %CO-30 | 104±1 | 53±6 | 46±5 | 8±4 | 35±7 | 47±7 | 35±8 | 55±5 |
| %CO-40 | 106±1 | 55±6 | 49±5 | 13±6 | 42±7 | 48±7 | 40±7 | 58±5 |
| %LVMW-10 | 102±1 | 17±7 | 13±4 | 5±3 | 24±7 | 17±9 | 21±7 | 9±4 |
| %LVMW-20 | 104±1 | 48±6 | 41±5 | 5±3 | 30±7 | 42±8 | 32±8 | 53±6 |
| %LVMW-30 | 105±1 | 58±6 | 50±5 | 8±5 | 38±7 | 51±8 | 38±8 | 61±6 |
| %LVMW-40 | 107±1 | 60±6 | 53±5 | 14±6 | 46±7 | 52±8 | 43±8 | 64±5 |
| %LVSP-10 | 101±0 | 56±7 | 55±8 | 34±5 | 62±9 | 52±11 | 63±7 | 40±8 |
| %LVSP-20 | 101±0 | 88±5NS | 87±3 | 36±5 | 73±6 | 83±7 | 73±7 | 87±2 |
| %LVSP-30 | 102±1 | 95±2 | 92±2 | 43±6 | 81±4 | 87±6 | 79±6 | 90±1 |
| %LVSP-40 | 102±1 | 95±1 | 92±2 | 54±7 | 86±3 | 89±5 | 83±4 | 91±1 |
| %LVDP-10 | 103±1 | 133±6 | 149±9 | 170±20 | 149±15 | 141±14 | 152±20 | 159±10 |
| %LVDP-20 | 106±2 | 120±4 | 121±7NS | 171±17 | 144±16 | 132±14NS | 147±12 | 132±11 |
| %LVDP-30 | 106±2 | 109±4NS | 110±9NS | 174±17 | 141±12 | 125±15NS | 134±11 | 131±10 |

| | | | | | | | | |
|---|---|---|---|---|---|---|---|---|
| %LVDP-40 | 105±3 | 114±3 | 107±10NS | 167±15 | 134±12 | 125±15NS | 137±9 | 133±10 |
| %LVdP/dT-10 | 101±0 | 51±8 | 55±10 | 23±7 | 54±11 | 45±11 | 57±8 | 41±11 |
| %LVdP/dT-20 | 102±1 | 85±6 | 87±4 | 23±6 | 66±7 | 83±9 | 67±8 | 86±3 |
| %LVdP/dT-30 | 102±1 | 94±2 | 92±3 | 33±7 | 76±5 | 86±7 | 74±6 | 87±2 |
| %LVdP/dT-40 | 102±1 | 94±2 | 92±3 | 42±8 | 81±3 | 88±6 | 79±5 | 88±2 |
| %ASP-10 | 100±0 | 52±6 | 50±7 | 38±5 | 59±9 | 48±10 | 60±7 | 44±7 |
| %ASP-20 | 99±0 | 80±4 | 78±3 | 47±3 | 70±5 | 77±7 | 69±6 | 82±2 |
| %ASP-30 | 98±1 | 86±2 | 82±2 | 47±5 | 76±4 | 80±6 | 75±4 | 85±1 |
| %ASP-40 | 98±0 | 86±2 | 83±2 | 58±6 | 80±2 | 82±4 | 78±3 | 86±1 |
| %MAP-10 | 100±0 | 79±7 | 80±10NS | 68±8 | 87±10 | 74±12 | 90±7NS | 72±9 |
| %MAP-20 | 101±0 | 106±4NS | 108±2 | 87±6 | 105±4NS | 105±7 | 100±6NS | 112±1 |
| %MAP-30 | 100±0 | 110±1 | 110±2 | 81±7 | 107±4NS | 106±4NS | 107±3 | 111±1 |
| %MAP-40 | 101±1 | 109±1 | 111±1 | 97±8NS | 112±2NS | 109±2NS | 109±2 | 110±1 |
| %CVR-10 | 97±1 | 189±22 | 149±21 | 388±103 | 171±31 | 188±35 | 184±27 | 160±20 |
| %CVR-20 | 96±2 | 146±8 | 144±8 | 635±135 | 260±61 | 164±19 | 239±54 | 145±5 |
| %CVR-30 | 95±2 | 154±8 | 143±9 | 399±55 | 210±27 | 164±22 | 226±51 | 150±6 |
| %CVR-40 | 97±2 | 157±9 | 146±9 | 319±41NS | 191±26 | 160±21 | 212±48 | 148±6 |
| %RPP-10 | 101±0 | 56±7 | 54±7 | 34±5 | 62±9 | 52±11 | 63±7 | 40±8 |
| %RPP-20 | 101±0 | 88±5NS | 86±3 | 36±5 | 73±6 | 83±7 | 72±7 | 87±2 |
| %RPP-30 | 101±1 | 95±2 | 90±3 | 42±6 | 80±4 | 87±6 | 78±6 | 90±1 |
| %RPP-40 | 102±1 | 95±1 | 91±2 | 54±7 | 85±3 | 89±5 | 83±4 | 91±1 |
| %EFFI-10 | 102±1 | 22±6 | 20±5 | 11±4 | 30±7 | 22±9 | 28±7 | 17±5 |
| %EFFI-20 | 103±1 | 51±6 | 46±4 | 11±3 | 36±7 | 47±8 | 38±8 | 61±6 |
| %EFFI-30 | 103±1 | 60±6 | 55±5 | 14±6 | 44±7 | 56±8 | 44±8 | 68±6 |
| %EFFI-40 | 105±1 | 63±6 | 58±5 | 19±7 | 52±7 | 57±8 | 49±8 | 70±5 |
| Rec-t | 0±0 | 14±2 | 14±2 | 27±11 | 18±4 | 13±2 | 16±8 | 13±1 |

By way of example, and not limitation, contemplated examples will now be given.

Example One

This contemplated example demonstrates the use of the composition and method of the present invention as a therapy for a patient having ischemic cardiac tissue.

A middle-aged male patient is admitted to intensive care following an acute myocardial infarction.

A thrombolytic drug was immediately administered intravenously to the patient. As soon as the drug infusion was completed, an infusion of substrates - amino acids and dextrose - was started.

The substrate mixture was prepared as follows. Five hundred mL of 10% dextrose was admixed with 200 mL of 10% Travasol®; the admixture was brought to one Liter with water. The final concentration of substrates was thus 5% dextrose and 2% amino acids (w:v). Selected electrolytes were added as prescribed by the attending physician. This admixture was infused via the same site as the thrombolytic drug, at a rate of 70 mL/hour until the infusion was complete.

During the above period the patient was managed as follows. The heart was monitored by continuous EKG recording. Serial blood samples were taken at 1-1.5 hour intervals, for the measurement of creatine phosphokinase (CPK). The enzyme, CPK, is released from damaged heart cells, and is a rough index of the

10

degree of injury severity and recovery.

It was observed that the EKG showed minimal evidence of reperfusion arrythmias, with a return to a reasonably normal pattern in 6-7 hours. Other patients with attacks of similar severity, but not treated with substrates, usually showed substantial evidence of reperfusion arrythmias and a return to acceptable patterns only after 8-10 hours.

Blood levels of CPK would have been expected to peak at about 10 hours; in this patient it peaked at about 8 hours.

The patient was discharged from the ICU in 2 days, and from the hospital 2 days thereafter. Subsequent testing showed recovery of the damaged myocardium to a greater degree than would have been seen in patients not infused with substrate.

Example Two

This contemplated example demonstrates the use of the composition and method of the present invention as a therapy prior to an ischemic event.

A middle-aged male underwent an angioplasty. A balloon catheter was placed so as to be surrounded by atheroma in an artery leading to the heart. The balloon was expanded to push the atheroma into the intima. The balloon was then partially deflated, moved downstream to the next section of atheroma, and the procedure repeated. The total time of near-total interruption of flow through this vessel, due to the angioplasty procedure per se, was one hour and 20 minutes.

Immediately upon removing the balloon portion of the catheter an infusion consisting of metabolic substrates was begun, through the lumen of the catheter. It was prepared as described above in Example One, except that the final concentration of dextrose was 10%; and of amino acids, 3%. In this manner, the heart was rather directly perfused with substrate for 20 minutes. The entire catheter was then removed so as to allow blood flow to return to the heart. The substrate mixture was then infused via a peripheral vein and continued until the infusion was completed.

Considering the degree of atheromatous occlusion and the duration of the angioplasty procedure, clinical experience led the cardiologist to predict that the patient would have significant abnormalities of the EKG for several hours following the procedure. However, infusion of the substrate seemed to allow the heart to evolve to a reasonably normal EKG in less time than expected. Hemodynamic parameters also reflected a return to normal in an accelerated time. The patient experienced no problems and was discharged that evening.

It should be understood that various changes and modifications to the presently preferred embodiments described herein will be apparent to those skilled in the art. Such changes and modifications can be made without departing from the scope of the present invention and without diminishing its attendant advantages. It is therefore intended that such changes and modifications be covered by the appended claims.

**Claims**

1. The use of amino acids for the preparation of a composition for treating ischemic cardiac tissue for aiding recovery of cardiac function in a patient having the ischemic cardiac tissue.

2. The use of amino acids for the preparation of a composition for prophylactically treating tissue susceptible to a cardiac ischemic event to limit damage from cardiac ischemia in a patient expected to have the cardiac ischemic event.

3. The use according to Claims 1 or 2 wherein the composition includes: arginine; leucine; isoleucine; lysine; valine; phenylalanine; histidine; threonine; methionine; tryptophan; alanine; proline; serine; tyrosine; amino acetic acid; wherein branched chain amino acids comprise 15% to 55% by weight of the amino acid content of the composition.

4. The use according to Claims 1 or 2 wherein the composition includes: isoleucine; leucine; and valine.

5. The use according to Claims 1 or 2 wherein the composition includes: arginine; leucine; isoleucine; lysine; valine; phenylalanine; histidine; threonine; methionine; tryptophan; alanine; proline; serine; tyrosine; and amino acetic acid.

6. The use according to Claims 1 or 2 wherein the composition is enriched in amino acids metabolized to $CO_2$ and $H_2O$ by pathways shared with those of the fatty acid oxidation.

7. The use according to Claims 1 or 2 wherein the composition includes at least one amino acid chosen from the group consisting of leucine, isoleucine, valine, methionine, phenylalanine, tyrosine, and tryptophan.

8. The use according to Claims 1 or 2 wherein the composition includes amino acids that supply intermediates to the tricarboxylic acid ("KREBS") cycle.

9. The use according to Claims 1 or 2 wherein the composition includes at least one amino acid chosen from the group consisting of glutamic acid, glutamine, aspartic acid, and asparagine.

10. The use according to Claims 1 or 2 wherein the composition includes: glutamic acid; aspartic acid; arginine; leucine; isoleucine; lysine; valine; phenylalanine; histidine; threonine; methionine; tryptophan; alanine; proline; serine; tyrosine; and amino acetic acid.

11. The use according to any of the preceding claims wherein the composition is provided for enteral administration.

12. The use according to any of the preceding claims wherein the composition is provided for parenteral administration.

13. Use of amino acids for the preparation of a composition that is administered to a patient during a surgical intervention for treating atherosclerosis.

14. The use according to Claim 13 wherein the surgical intervention is percutaneous transluminal coronary angioplasty.

15. The use according to Claim 13 wherein the surgical intervention is coronary by-pass surgery.

**Patentansprüche**

1. Verwendung von Aminosäuren zur Herstellung einer Zusammensetzung zur Behandlung eines ischämischen Herzgewebes zur Unterstützung der Erholung der Herzfunktion bei einem Patienten mit einem ischämischen Herzgewebe.

2. Verwendung von Aminosäuren zur Herstellung einer Zusammensetzung zur prophylaktischen Behandlung von Gewebe, das für einen ischämischen Herzanfall anfällig ist, um den Schaden durch eine Herz-Ischämie bei einem Patienten zu begrenzen, von dem zu erwarten ist, daß er den ischämischen Herzanfall erleidet.

3. Verfahren nach den Ansprüchen 1 oder 2, bei dem die Zusammensetzung einschließt: Arginin; Leucin; Isoleucin; Lysin; Valin; Phenylalanin; Histidin; Threonin; Methionin; Tryptophan; Alanin; Prolin; Serin; Tyrosin; Aminoessigsäure, wobei verzweigt-kettige Aminosäuren 15 bis 55 Gew.-% des Aminosäuregehalts der Zusammensetzung bilden.

4. Verwendung nach den Ansprüchen 1 oder 2, bei der die Zusammensetzung einschließt: Isoleucin; Leucin; und Valin.

5. Verwendung nach den Ansprüchen 1 oder 2, bei der die Zusammensetzung einschließt: Arginin; Leucin; Isoleucin; Lysin; Valin; Phenylalanin; Histidin; Threonin; Methionin; Tryptophan; Alanin; Prolin; Serin; Tyrosin; und Aminoessigsäure.

6. Verwendung nach den Ansprüchen 1 oder 2, bei der die Zusammensetzung im Hinblick auf Aminosäuren angereichert ist, die zu $CO_2$ und $H_2O$ auf Abbauwegen metabolisiert werden, die denen der Fettsäureoxidation gemeinsam sind.

**7.** Verwendung nach den Ansprüchen 1 oder 2, bei der die Zusammensetzung wenigstens eine Aminosäure einschließt, die aus der Gruppe ausgewählt ist, die aus Leucin, Isoleucin, Valin, Methionin, Phenylalanin, Tyrosin und Tryptophan besteht.

**8.** Verwendung mach den Ansprüchen 1 oder 2, bei der die Zusammensetzung Aminosäuren einschließt, die Zwischenstufen für den Tricarbonsäure-("Krebs")-Zyklus liefern.

**9.** Verwendung nach den Ansprüchen 1 oder 2, bei der die Zusammensetzung wenigstens eine Aminosäure einschließt, die aus der Gruppe gewählt ist, die aus Glutaminsäure, Glutamin, Asparaginsäure und Asparagin besteht.

**10.** Verwendung nach den Ansprüchen 1 oder 2, bei der die Zusammensetzung einschließt: Glutaminsäure; Asparaginsäure; Arginin; Leucin; Isoleucin; Lysin, Valin; Phenylalanin; Histidin; Threonin; Methionin; Tryptophan; Alanin; Prolin; Serin; Tyrosin; und Aminoessigsäure.

**11.** Verwendung nach irgendeinem der vorausgehenden Ansprüche, bei der die Zusammensetzung für eine enterale Verabreichung bereitgestellt wird.

**12.** Verwendung nach irgendeinem der vorausgehenden Ansprüche, bei der die Zusammensetzung für eine parenterale Verabreichung bereitgestellt wird.

**13.** Verwendung von Aminosäuren zur Herstellung einer Zusammensetzung, die einem Patienten während eines chirurgischen Eingriffs zur Behandlung der Atherosklerose verabreicht wird.

**14.** Verwendung nach Anspruch 13, bei der der chirurgische Eingriff eine perkutane transluminale Koronar-Gefäßplastik ist.

**15.** Verwendung nach Anspruch 13, bei der der chirurgische Eingriff eine koronare Bypass-Operation ist.

**Revendications**

**1.** Utilisation d'aminoacides pour la préparation d'une composition destinée au traitement d'un tissu cardiaque ischémique pour faciliter le rétablissement de la fonction cardiaque chez un patient possédant un tel tissu cardiaque ischémique.

**2.** Utilisation d'aminoacides pour la préparation d'une composition destinée au traitement prophylactique d'un tissu susceptible de subir un événement ischémique cardiaque pour limiter l'endommagement provenant de l'ischémie cardiaque chez un patient exposé à présenter cet événement ischémique cardiaque.

**3.** Utilisation suivant la revendication 1 ou 2, dans laquelle la composition comprend : de l'arginine ; de la leucine ; de l'isoleucine ; de la lysine ; de la valine ; de phénylalanine ; de l'histidine ; de la thréonine ; de la méthionine ; du tryptophane ; de l'alanine ; de la proline de la sérine ; de la tyrosine, de l'acide aminoacétique ; les aminoacides à chaîne ramifiée représentant 15 % à 55 % en poids de la teneur en aminoacides de la composition.

**4.** Utilisation suivant la revendication 1 ou 2, dans laquelle la composition comprend : de l'isoleucine ; de la leucine et de la valine.

**5.** Utilisation suivant la revendication 1 ou 2, dans laquelle la composition comprend : de l'arginine ; de la leucine ; de l'isoleucine ; de la lysine ; de la valine ; de phénylalanine ; de l'histidine ; de la thréonine ; de la méthionine ; du tryptophane ; de l'alanine ; de la proline ; de la sérine ; de la tyrosine, de l'acide aminoacétique.

**6.** Utilisation suivant la revendication 1 ou 2, dans laquelle la composition est enrichie en aminoacides métabolisés en $CO_2$ et $H_2O$ par des voies métaboliques en commun avec celles de l'oxydation des acides gras.

**7.** Utilisation suivant la revendication 1 ou 2, dans laquelle la composition comprend au moins un aminoacide choisi dans le groupe consistant en leucine, isoleucine, valine, méthionine, phénylalanine, tyrosine et tryptophane.

**8.** Utilisation suivant la revendication 1 ou 2, dans laquelle la composition comprend des aminoacides qui fournissent des intermédiaires au cycle des acides tricarboxyliques ("cycle de KREBS").

**9.** Utilisation suivant la revendication 1 ou 2, dans lequel la composition comprend au moins un aminoacide choisi dans le groupe consistant en acide glutamique, glutamine, acide aspartique et asparagine.

**10.** Utilisation suivant la revendication 1 ou 2, dans laquelle la composition comprend : de l'acide glutamique, de l'acide aspartique, de l'arginine, de la leucine, de l'isoleucine, de la lysine, de la valine, de la phénylalanine, de l'histidine, de la thréonine, de la méthionine, du tryptophane, de l'alanine, de la proline, de la sérine, de la tyrosine et de l'acide aminoacétique.

**11.** Utilisation suivant l'une quelconque des revendications précédentes, dans laquelle la composition est fournie pour l'administration entérale.

**12.** Utilisation suivant l'une quelconque des revendications précédentes, dans laquelle la composition est fournie pour une administration parentérale.

**13.** Utilisation d'aminoacides pour la préparation d'une composition qui est administrée à un patient au cours d'une intervention chirurgicale pour le traitement de l'athérosclérose.

**14.** Utilisation suivant la revendication 13, dans laquelle l'intervention chirurgicale consiste en une angioplastie coronarienne transluminale percutanée.

**15.** Utilisation suivant la revendication 13, dans laquelle l'intervention chirurgicale est une intervention chirurgicale de pontage coronarien.